# EUROPEAN PATENT SPECIFICATION

(11) **EP 3 862 436 B1**
(45) Date of publication and mention of the grant of the patent: **28.08.2024**
(21) Application number: 19869444.0
(22) Date of filing: 02.10.2019
(51) Int. Cl.: G01N 33/58, G01N 33/68

(54) **METHOD FOR PREDICTING OR PROGNOSTICATING THE RESPONSE TO THE TREATMENT OF MULTIPLE SCLEROSIS WITH INTERFERON BETA**
VERFAHREN ZUR VORHERSAGE ODER PROGNOSTIZIERUNG DES ANSPRECHENS AUF DIE BEHANDLUNG VON MULTIPLER SKLEROSE MIT INTERFERON BETA
MÉTHODE POUR PRÉDIRE OU PRONOSTIQUER LA RÉPONSE AU TRAITEMENT DE LA SCLÉROSE EN PLAQUES AVEC L'INTERFÉRON BÊTA

(30) Priority: 02.10.2018 ES 201830953
(43) Date of publication of application: 11.08.2021
(73) Proprietor: Servicio Andaluz de Salud, 41071 Sevilla (ES); Universidad de Málaga, 29071 Málaga (ES)
(72) Inventor: OLIVER MARTOS, Begoña, 29010 Málaga (ES); LEYVA FERNÁNDEZ, Laura, 29010 Málaga (ES); HURTADO GUERRERO, Isaac, 29010 Málaga (ES); FERNÁNDEZ FERNÁNDEZ, Óscar, 29010 Málaga (ES); PAVÍA MOLINA, José, 29071 Málaga (ES)
(74) Representative: Hoffmann Eitle
(86) International application number: PCT/ES2019/070660
(87) International publication number: WO 2020/070363

(56) References cited:
- EP-A1- 2 930 183
- WO-A1-2015/140793
- WO-A1-2015/140793
- WO-A2-2010/010057
- GILLI F. ET AL: "Expression and regulation of IFNalpha/beta receptor in IFNbeta-treated patients with multiple sclerosis", NEUROLOGY, vol. 71, no. 24, 9 December 2008 (2008-12-09), US, pages 1940 - 1947, XP55939852, ISSN: 0028-3878, Retrieved from the Internet <URL:https://n.neurology.org/content/neurology/71/24/1940.full-text.pdf> DOI: 10.1212/01.wnl.0000327340.50284.8d
- GILLI F. ET AL: "Expression and regulation of IFNalpha/beta receptor in IFNbeta-treated patients with multiple sclerosis", NEUROLOGY, vol. 71, no. 24, 9 December 2008 (2008-12-09), US, pages 1940 - 1947, XP055939852, ISSN: 0028-3878, Retrieved from the Internet <URL:https://n.neurology.org/content/neurology/71/24/1940.full-text.pdf> DOI: 10.1212/01.wnl.0000327340.50284.8d
- ÓRPEZ-ZAFRA T ET AL: "Development and validation of an ELISA for quantification of soluble IFN-beta receptor: assessment in multiple sclerosis", BIOANALYSIS, FUTURE SCIENCE, LONDON, UK, vol. 7, no. 22, 1 January 2015 (2015-01-01), pages 2869 - 2880, XP002756854, ISSN: 1757-6180, DOI: 10.4155/BIO.15.208
- ORPEZ-ZAFRA TERESA ET AL: "Decreased soluble IFN-beta receptor (sIFNAR2) in multiple sclerosis patients: A potential serum diagnostic biomarker", MULTIPLE SCLEROSIS JOURNAL, vol. 23, no. 7, 31 May 2017 (2017-05-31), US, pages 937 - 945, XP055805559, ISSN: 1352-4585, DOI: 10.1177/1352458516667564
- M.J. PINTO-MEDEL ET AL: "Evaluation of soluble IFNß receptor (sIFNAR2) as IFNß response biomarker in multiple sclerosis patients", MULTIPLE SCLEROSIS JOURNAL, vol. 24, no. S2, 9 October 2018 (2018-10-09), US, pages 1 - 2, XP055700014, ISSN: 1352-4585, DOI: 10.1177/1352458518798590
- SUARDÍAZ M ET AL: "Recombinant soluble IFN receptor (sIFNAR2) exhibits intrinsic therapeutic efficacy in a murine model of Multiple Sclerosis", NEUROPHARMACOLOGY, ELSEVIER, AMSTERDAM, NL, vol. 110, 22 July 2016 (2016-07-22), pages 480 - 492, XP029733846, ISSN: 0028-3908, DOI: 10.1016/J.NEUROPHARM.2016.07.026
- ÓRPEZ-ZAFRA T, PAVÍA J, PINTO-MEDEL M J, HURTADO-GUERRERO I, RODRIGUEZ-BADA J L, MARTÍN MONTAÑEZ E, FERNÁNDEZ Ó, LEYVA L, OLIVER-M: "Development and validation of an ELISA for quantification of soluble IFN-beta receptor: assessment in multiple sclerosis", BIOANALYSIS, vol. 7, no. 22, 1 January 2015 (2015-01-01), pages 2869 - 2880, XP002756854, ISSN: 1757-6180, DOI: 10.4155/bio.15.208
- TERESA ÓRPEZ-ZAFRA, JOSE PAVÍA, ISAAC HURTADO-GUERRERO, MARIA J PINTO-MEDEL, JOSE LUIS RODRIGUEZ BADA, PATRICIA URBANEJA, MARGARIT: "Decreased soluble IFN-beta receptor (sIFNAR2) in multiple sclerosis patients: A potential serum diagnostic biomarker", MULTIPLE SCLEROSIS JOURNAL JUN 2017, vol. 23, no. 7, 31 May 2017 (2017-05-31), pages 937 - 945, XP055805559, ISSN: 1352-4585, DOI: 10.1177/1352458516667564
- RÍO J, CASTILLÓ J, ROVIRA A, TINTORÉ M, SASTRE-GARRIGA J, HORGA A, NOS C, COMABELLA M, AYMERICH X, MONTALBÁN X: "Measures in the first year of therapy predict the response to interferon beta in MS", MULTIPLE SCLEROSIS JOURNAL, vol. 15, no. 7, 1 July 2009 (2009-07-01), pages 848 - 853, XP055805565, ISSN: 1352-4585, DOI: 10.1177/1352458509104591
- M.J. PINTO-MEDEL, I. HURTADO GUERRERO, A. ALONSO, J.L. RODRIGUEZ-BADA, P. URBANEJA, Ó. FERNÁNDEZ, L. LEYVA1, B. OLIVER-MARTOS: "Evaluation of soluble IFNbeta receptor (sIFNAR2) as IFNbeta response biomarker in multiple sclerosis patients", MULTIPLE SCLEROSIS JOURNAL 20181001, vol. 24, no. S2, 1 October 2018 (2018-10-01), pages 1 - 2, XP055700014, ISSN: 1352-4585, DOI: 10.1177/1352458518798590

## Description

The present invention is comprised within the field of biomedicine, and relates to a method for predicting or prognosticating the response of multiple sclerosis patients to treatment with IFNβ.

### PRIOR STATE OF THE ART

Multiple sclerosis (MS) is a chronic inflammatory demyelinating disease of the central nervous system (CNS), presumably of autoimmune origin. It is characterized by the presence of inflammatory lesions, called plaques, in the white and grey matter of the CNS, in which myelin loss and a certain degree of axonal degeneration occur.

Although various drugs have been developed in recent years to alleviate the effects of this disease, interferon beta (IFNβ) still constitutes one of the first-line treatments in view of its efficacy and safety profile. It has been demonstrated in various clinical trials that IFNβ reduces the frequency and severity of flare ups, the number and volume of brain lesions observed by means of resonance, and progression in the expanded disability status scale. However, a significant percentage of patients (30-50%) do not respond adequately to the treatment as they continue to experience flare ups and to progress in the expanded disability status scale.

WO2015140793 discloses a method for predicting responsiveness of a subject with multiple sclerosis to interferon (IFN) treatment, in particular IFNβ, comprising determining the expression level of a plurality of genes comprising Janus Kinase 1 (JAK1), Interferon (Alpha, Beta And Omega) Receptor 2 (IFNAR2), SKI, or their combination, where a significant difference between the expression level of the genes in the subject sample, compared to a control value is indicative of the responsiveness of the subject to IFN treatment.

Although many treatment response biomarkers are in the research phase, none of them is validated and implemented in clinical practice to help in predicting whether a multiple sclerosis (MS) patient will respond adequately to treatment with IFNβ.

It is therefore necessary to provide a preferably minimally invasive and easy-to-perform test for determining whether or not a multiple sclerosis patient will respond to treatment with IFNβ.

### DESCRIPTION OF THE FIGURES

**Figure 1****.** Detection of recombinant slFNAR2 by means of Western blot (A) and standard ELISA curve (B) in mean values of optical density (OD) vs. concentration (Conc.).
**Figure 2****.** Determination of the serum concentration of slFNAR2 in untreated MS patients (UT-MS) and healthy controls (HC) by means of ELISA. Untreated MS patients showed sIFNAR2 levels significantly lower than HC (Orpez-Zafra *et al.,* 2015)
**Figure 3****.** UT-MS patients showed sIFNAR2 levels significantly lower than healthy controls (HC) and controls of other inflammatory neurological diseases (OIND), and showed no differences with the patients with clinically isolated syndrome (CIS). The heterogeneous group of OIND showed no significant differences with HC and their levels increased significantly when compared with patients treated with IFNβ (MS-IFNβ). Moreover, MS patients treated with IFNβ showed significantly higher slFNAR2 levels than MS patients without prior treatment and were significantly lower than those of HC.

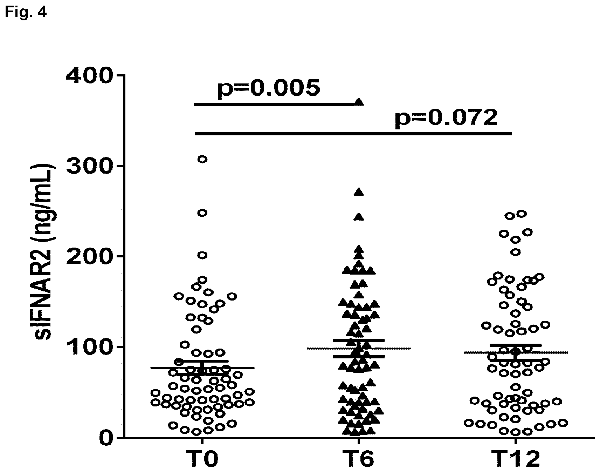

**Figure 4****.** Follow-up of treatment with IFNβ over time, at the start (T0), at 6 months (6T), and up to 12 months (T12). Treatment with IFNβ increases sIFNAR2 levels. With all the analyzed patients, IFNβ significantly increased sIFNAR2 levels after 6 months of starting the treatment.

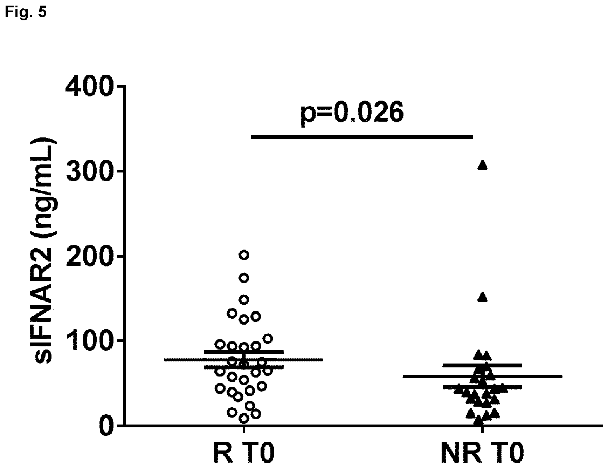

**Figure 5****.** Baseline slFNAR2 levels as an IFNβ response biomarker. Before the start of treatment (T0), patients classified as non-responders (NR) had significantly lower slFNAR2 levels in comparison with patients classified as responders (R). Logistic regression analysis showed that patients with baseline slFNAR2 levels below 43.2 ug/ml had an OR of 5.1 patients not responding to treatment with IFNβ. The model was fitted for possible.

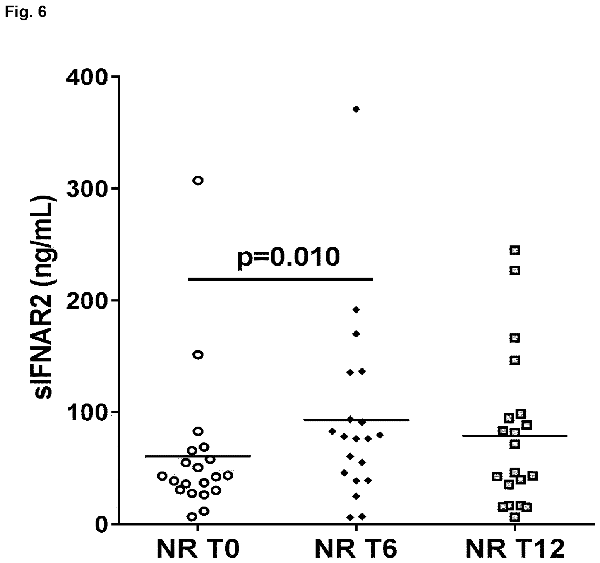

**Figure 6****.** Response to treatment with IFNβ in NR, at the start of treatment (T0), at 6 months (6T), and up to 12 months (T12). sIFNAR2 levels in these patients increased significantly after 6 months of treatment.

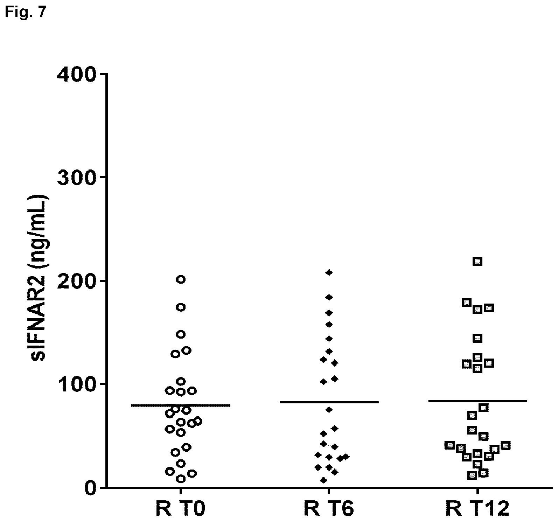

**Figure 7****.** Response to treatment with IFNβ in R, at the start of treatment (T0), at 6 months (6T), and up to 12 months (T12). sIFNAR2 levels in these patients remained stable after 6 months of treatment.

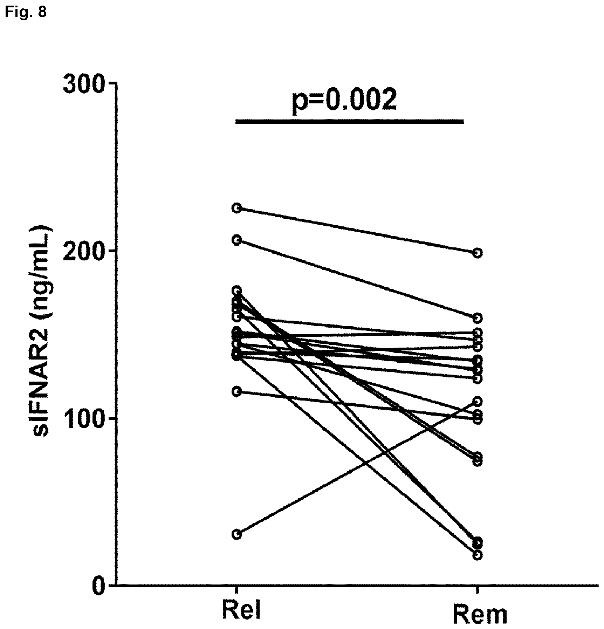

**Figure 8****.** Connection with other clinical variables. High slFNAR2 levels were observed in patients during relapse (Rel) in comparison with the same patient in remission (Rem) (p = 0.002).

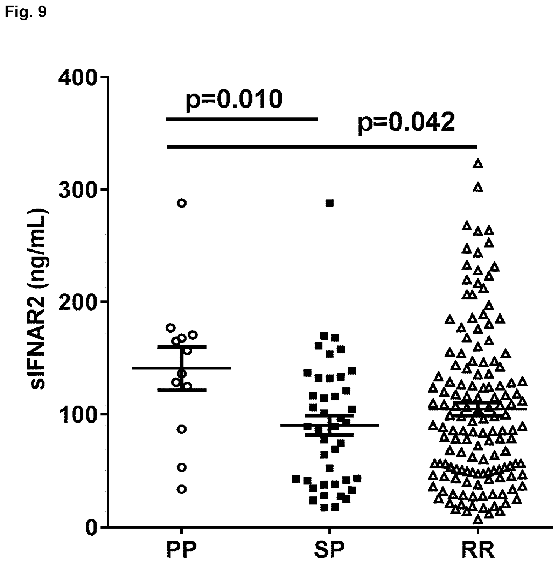

**Figure 9****.** Analysis of the evolutionary clinical form of multiple sclerosis from RR: relapsing-remitting; SP: secondary progressive; to PP: primary progressive. It was observed that slFNAR2 was elevated in PP in comparison with RR and SP (p = 0.042, p = 0.010).

### DESCRIPTION OF THE INVENTION

The authors of the present invention have evaluated serum slFNAR2 levels as an IFNβ response biomarker, as well as the connection thereof with other clinical variables, developing a method for predicting or prognosticating the response of multiple sclerosis patients to IFNβ. As shown in the examples of the present invention, the baseline sIFNAR2 levels can predict the response to treatment with IFNβ and may have clinical applicability when selecting treatment. The lower baseline sIFNAR2 levels in non-responder patients are restored after six months of treatment with IFNβ and reach values similar to responder patients.

In summary, the authors of the present invention propose the determination of a serum biomarker with a technique developed in their laboratory which allows predicting whether or not an MS patient will respond to treatment with IFNβ.

Among other advantages, the test is minimally invasive for the patient since it is performed in serum, and furthermore it is easy to implement since ELISA is a common tool used in clinical laboratories.

The invention is defined in the appended claims.

A **first aspect** disclosed relates to the use of *IFNAR2.3* expression product and/or sIFNAR2 levels, preferably serum levels, for predicting or prognosticating the response of an individual with multiple sclerosis to treatment with IFNβ.

Numerous transcription variants encoding at least three different isoforms have been identified for the gene encoding the IFNAR2 subunit of the IFNβ receptor. The amino acid sequence of slFNAR2 is found in GenBank (NCBI) with accession number L41943.1 and in SEQ ID NO: 2 in reference to gene *IFNAR2.3.* Said SEQ ID NO: 2 is represented by the following amino acid sequence:

(MLLSQNAFIFRSLNLVLMVYISLVFGISYDSPDYTDESCTFKISLRNFRSILSWELKNHSIVPTHY
TLLYTIMSKPEDLKVVKNCANTTRSFCDLTDEWRSTHEAYVTVLEGFSGNTTLFSCSHNFWLAI
DMSFEPPEFEIVGFTNHINVMVKFPSIVEEELQFDLSLVIEEQSEGIVKKHKPEIKGNMSGNFTYII
DKLIPNTNYCVSVYLEHSDEQAVIKSPLKCTLLPPGQESEFS).

In the context of the present disclosure, sIFNAR2 is also defined by a nucleotide or polynucleotide sequence which constitutes the coding sequence of the protein set forth in SEQ ID NO: 2 and would comprise different variants originating from:
a) nucleic acid molecules encoding a polypeptide comprising the amino acid sequence of SEQ ID NO: 2,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with at least 60%, 70%, 80%, 90%, 95%, 98%, or 99% identity with SEQ ID NO: 2, and in which the polypeptide encoded by said nucleic acids exhibits the activity and structural characteristics of the IFNAR2.3 protein. Said nucleic acid molecules include, among others, the molecule set forth in GenBank (NCBI) sequence L41943.1 and SEQ ID NO: 1. Said SEQ ID NO: 1 is represented by the following nucleotide sequence:

(agatgtaaaagtcaagagaagactctaaaaatagcaaagatgcttttgagccagaatgccttcatcttcagatcacttaatttggttctc
atggtgtatatcagcctcgtgtttggtatttcatatgattcgcctgattacacagatgaatcttgcactttcaagatatcattgcgaaatttccg
gtccatcttatcatgggaattaaaaaaccactccattgtaccaactcactatacattgctgtatacaatcatgagtaaaccagaagatttg
aaggtggttaagaactgtgcaaataccacaagatcattttgtgacctcacagatgagtggagaagcacacacgaggcctatgtcacc
gtcctagaaggattcagcgggaacacaacgttgttcagttgctcacacaatttctggctggccatagacatgtcttttgaaccaccagag
tttgagattgttggttttaccaaccacattaatgtgatggtgaaatttccatctattgttgaggaagaattacagtttgatttatctctcgtcattg
aagaacagtcagagggaattgttaagaagcataaacccgaaataaaaggaaacatgagtggaaatttcacctatatcattgacaag
ttaattccaaacacgaactactgtgtatctgtttatttagagcacagtgatgagcaagcagtaataaagtctcccttaaaatgcaccctcct
tccacctggccaggaatcagaattttcataactttttagcctggccatttcctaacctgccaccgttggaagccatggatatggtggaggt
catttacatcaacagaaagaagaaagtgtgggattataattatgatgatgaaagtgatagcgatactgaggcagcgcccaggacaa
gtggcggtggctataccatgcatggactgactgtcaggcctctgggtcaggcctctgccacctctacagaatcccagttgatagacccg
gagtccgaggaggagcctgacctgcctgaggttgatgtggagctccccacgatgccaaaggacagccctcagcagttggaactctt
gagtgggccctgtgagaggagaaagagtccactccaggacccttttcccgaagaggactacagctccacggaggggtctgggggc
agaattaccttcaatgtggacttaaactctgtgtttttgagagttcttgatgacgaggacagtgacgacttagaagcccctctgatgctatc
gtctcatctggaagagatggttgacccagaggatcctgataatgtgcaatcaaaccatttgctggccagcggggaagggacacagc
caacctttcccagcccctcttcagagggcctgtggtccgaagatgctccatctgatcaaagtgacacttctgagtcagatgttgaccttgg
ggatggttatataatgagatgactccaaaactattgaatgaacttggacagacaagcacctacagggttctttgtctctgcatcctaactt
gctgccttatcgtctgcaagtgttctccaagggaaggaggaggaaactgtggtgttcctttcttccaggtgacatcacctatgcacattcc

cagtatggggaccatagtatcattcagtgcattgtttacatattcaaagtggtgcactttgaaggaagcacatgtgcacctttcctttacact
aatgcacttaggatgtttctgcatcatgtctaccagggagcagggttccccacagtttcagaggtggtccaggaccctatgatatttctctt
ctttcgttcttttttttttttttttgagacagagtctcgttctgtcgcccaagctggagcgcaatggtgtgatcttggctcactgcaacatccgcct
cccgggttcaggtgattctcctgcctcagcctccctcgcaagtagctgggattacaggcgcctgccaccatgcctagcaaatttttgtattt
ttagtggagacaggattttaccatgttggccaggctggtctcgaactcctgacctcaagtgatctgccctcctcagcctcgtaaagtgctg
ggattacaggggtgagccgctgtgcctggctggccctgtgatatttctgtgaaataaattgggccagggtgggagcagggaaagaaa
aggaaaatagtagcaagagctgcaaagcaggcaggaagggaggaggagagccaggtgagcagtggagagaaggggggccc
tgcacaaggaaacagggaagagccatcgaagtttcagtcggtgagccttgggcacctcacccatgtcacatcctgtctcctgcaattg
gaattccaccttgtccagccctccccagttaaagtggggaagacagactttaggatcacgtgtgtgactaatacagaaaggaaacat
ggcgtcggggagagggataaaacctgaatgccatattttaagttaaaaaaaaaaaa).

A **second aspect** disclosed relates to a method for obtaining data useful for predicting or prognosticating the response of an individual with multiple sclerosis to treatment with IFNβ, hereinafter the first method of the invention, which comprises:
a) measuring *IFNAR2.3* expression product and/or slFNAR2 levels in a biological sample isolated from the individual, and
b) comparing the levels obtained in step (a) with a reference amount.

A first aspect of the invention relates to a method for predicting or prognosticating the response of an individual with multiple sclerosis to treatment with IFNβ which comprises:
a) measuring slFNAR2 protein levels in a serum sample isolated from the individual, and
b) comparing the levels obtained in step (a) with a reference amount, and
c) assigning the individual of step (a) to the group of non-responder individuals when he or she has significantly lower slFNAR2 protein levels in serum in comparison with responder individuals.

The reference amount is obtained from the constitutive expression values of *IFNAR2.3* in a group of individuals responding to multiple sclerosis treatment with IFNβ. The suitable reference amounts can be determined by means of the method of the present invention from a reference sample that can be analyzed, for example, simultaneously or consecutively, together with the test biological sample. In that sense, for example, but without limitation, the reference sample can be negative controls, i.e., the amounts detected by means of the method of the invention in samples from individuals not responding to multiple sclerosis treatment with IFNβ. In the invention, *IFNAR2.3* expression product is the slFNAR2 protein. In another more preferred embodiment, the invention comprises comparing the detection of the slFNAR2 protein in the biological sample of (a) with the detection of the slFNAR2 protein in a reference population.

The methods of the invention for identifying individuals with multiple sclerosis responding and not responding to treatment with IFNβ are preferably carried out before starting treatment with IFNβ.

As it is used herein, the term "comparing" refers, but is not limited, to comparing *IFNAR2.3* expression products and/or slFNAR2 levels in a test sample with the reference population, or alternatively, comparing the amount of gene expression products, or the amount of anti-IFNAR2.3 antibodies, and/or slFNAR2 in the biological sample to be analyzed, also referred to as test biological sample, with an amount of gene expression products, or with an amount of anti-IFNAR2.3 antibodies, and/or slFNAR2 levels in one or more desirable reference samples. The reference sample can be analyzed, for example, simultaneously or consecutively, together with the test biological sample. The comparison described in part (c) of the method of the present invention can be performed by hand or with the help of a computer.

Steps (b) and/or (c) of the methods described above can be completely or partially automated, for example, by means of robotic sensing equipment for detecting the amount in step (b) or performing a computerized comparison in step (c).

The method is an *in vitro* method and the sample on which parameters are measured is an isolated sample. In that sense, an "isolated biological sample" includes, but is not limited to, cells, tissues, and/or biological fluids of an organism, obtained by means of any method known by one skilled in the art. In the invention, the biological sample isolated from an individual in step (a) is serum. In another aspect (not claimed), the biological sample isolated from an individual in step (a) is cerebrospinal fluid.

As it is used herein, the term "individual" refers to animals, preferably mammals, and more preferably humans. The term "individual" is not intended to be limiting in any aspect, where the individual can be of any age and sex, and can be in any physical condition.

As they are understood herein, "slFNAR2" levels refer to the *IFNAR2.3* gene expression product, preferably being the amino acid sequence of sIFNAR2.

Although the measurement of sIFNAR2 levels can be qualitative, the amount or the concentration can also be determined in a quantitative or semi-quantitative manner and can be carried out directly or indirectly. Direct measurement refers to the measurement of the amount or concentration of the gene expression product, based on a signal directly obtained from the detection of the protein. Said signal, which can also be referred to as intensity signal, can be obtained, for example, by measuring an intensity value of a chemical or physical property of said products. Indirect measurement includes the measurement obtained from a secondary component or a biological measurement system (for example, the measurement of cell responses, ligands, "labels," or enzymatic reaction products).

The detection of *IFNAR2.3* expression product and/or slFNAR2 levels can be performed by any means known in the state of the art.

In another preferred embodiment, the detection of the amount of sIFNAR2 levels is performed by means of an immunoassay. As it is used herein, the term "immunoassay" refers to any analytical technique which is based on an antibody-antigen conjugation reaction. Examples of immunoassays known in the state of the art are, for example, but without limitation: immunoblot, enzyme-linked immunosorbent assay (ELISA), line immunoassay (LIA), radioimmunoassay (RIA), immunofluorescence, x-map, or protein chips.

In another preferred embodiment, the immunoassay is an enzyme-linked immunosorbent assay or ELISA. ELISA is based on the premise that an immunoreagent (antigen or antibody) can be immobilized on a solid support, with said system then being contacted with a fluid phase containing the complementary reagent which can bind to a marker compound. There are different types of ELISA: direct ELISA, indirect ELISA, or sandwich ELISA. In a preferred embodiment of this aspect of the invention, the ELISA is a sandwich ELISA.

As it is used herein, the term "marker compound" refers to a compound capable of producing a chromogenic, fluorogenic, radioactive, and/or chemiluminescent signal allowing the detection and quantification of the amount of anti-IFNAR2.3 antibodies. The marker compound is selected from the list comprising radioisotopes, enzymes, fluorophores, or any molecule that can be conjugated with another molecule or directly detected and/or quantified. This marker compound can bind to the antibody directly or through another compound. Some examples of directly binding marker compounds are, but without limitation, enzymes such as alkaline phosphatase or peroxidase, radioactive isotopes such as ³²P or ³⁵S, fluorochromes such as fluorescein or metal particles, for direct detection by means of colorimetry, autoradiography, fluorimetry, or metallography, respectively.

### DIAGNOSTIC KIT OR DEVICE AND USES

Another aspect of the present invention relates to the use of a kit comprising the elements needed for quantifying slFNAR2 protein levels in serum, for predicting or prognosticating the response of an individual with multiple sclerosis to treatment with IFNβ; wherein said kit comprises at least one anti-slFNAR2 antibody.

In a preferred embodiment, the kit comprises secondary antibodies or positive and/or negative controls. In a much more preferred embodiment, the kit comprises the polypeptide used in the invention, produced by means of recombinant technology, as a positive control. The kit can furthermore include, without any type of limitation, buffers, protein extraction solutions, agents for preventing contamination, protein degradation inhibitors, etc.

Moreover, the kit may include all the supports and containers needed for optimizing and putting it in operation. Preferably, the kit further comprises instructions for carrying out the methods of the invention.

In another preferred embodiment, the kit comprises:
a) a solid support with a primary antibody attached thereto
b) secondary antibody
c) a solution containing an enzymatic marker-labeled detection antibody;
d) a reagent.

In an even more preferred embodiment, the primary antibody is an antibody comprising the amino acid sequence of SEQ ID NO: 3

(MLLSQNAFIVRSLNLVLMVYISLVFGISYDSPDYTDESCTFKISLRNFRSILSWELKNHSIVPTHY
TLLYTIMSKPEDLKVVKNCANTTRSFCDLTDEWRSTHEAYVTVLEGFSGNTTLFSCSHNFWLAI
DMSFEPPEFEIVGFTNHINVMVKFPSIVEEELQFDLSLVIEEQSEGIVKKHKPEIKGNMSGNFTYII
DKLIPNTNYCVSVYLEHSDEQAVIKSPLKCTLLPPGQESESAESAKIGGIITVFLIALVLTSTIVTLK
WIGYICLRNSLPKVLRQGLTKGWNAVAIHRCSHNALQSETPELKQSSCLSFPSSWDYKRASLCP
SD).

In another more preferred embodiment, the secondary antibody is an antibody comprising the amino acid sequence of SEQ ID NO: 4

(MLLSQNAFIVRSLNLVLMVYISLVFGISYDSPDYTDESCTFKISLRNFRSILSWELKNHSIVPTHY
TLLYTIMSKPEDLKVVKNCANTTRSFCDLTDEWRSTHEAYVTVLEGFSGNTTLFSCSHNFWLAI
DMSFEPPEFEIVGFTNHINVMVKFPSIVEEELQFDLSLVIEEQSEGIVKKHKPEIKGNMSGNFTYII
DKLIPNTNYCVSVYLEHSDEQAVIKSPLKCTLLPPGQESESAESAKIGGIITVFLIALVLTSTIVTLK
WIGYICLRNSLPKVLRQGLTKGWNAVAIHRCSHNALQSETPELKQSSCLSFPSSWDYKRASLCP
SD)

### MEDICAL USES

Individuals with multiple sclerosis identified as IFNβ responders according to the methods of the invention can therefore be treated with IFNβ.

Therefore, another aspect described relates to a method for classifying individuals with multiple sclerosis into two groups, a first group including individuals identified as IFNβ responders and a second group including individuals identified as IFNβ non-responders.

Another aspect described relates to IFNβ for use in the treatment of multiple sclerosis in an individual classified as a responder according to any of the methods of the invention.

### AUTOMATION OF THE METHOD OF THE INVENTION BY IMPLEMENTING IT IN A COMPUTER PROGRAM

Another **aspect** described relates to a computer program comprising program instructions to cause a computer to carry out into practice the method according to any of the methods of the invention.

In particular, the disclosure encompasses computer programs disposed on or within a carrier.

The carrier can be any entity or device capable of supporting the program. When the program is incorporated in a signal that can be transported directly through a cable or another device or means, the carrier can be formed by said cable or another device or means. As a variant, the carrier could be an integrated circuit in which the program is included, the integrated circuit being adapted to run, or to be used for running, the corresponding processes.

For example, the programs could be incorporated in a storage medium, such as a ROM, a CD ROM, or a semiconductor ROM, a USB memory, or a magnetic recording support, for example, a floppy disk or a hard disk. Alternatively, the programs could be supported in a transmissible carrier signal. For example, the signal could be an electric or optical signal that could be transported by electrical or optical cable, radio, or any other means.

The disclosure also covers computer programs adapted for any processing means to be able to carry out into practice the methods of the invention. Such programs can be in the form of a source code, an object code, an intermediate code and object code source, such as, for example, in the partially compiled form or in any other form suitable for use in putting the processes according to the invention into practice. The computer programs also encompass cloud applications based on said method.

Another aspect described relates to a computer-readable storage medium comprising program instructions capable of causing a computer to carry out the steps of any of the methods of the invention.

Another aspect described relates to a transmissible signal comprising program instructions capable of causing a computer to carry out the steps of any of the methods of the invention.

The first and/or second methods of the invention may include additional steps such as, for example, separation of proteins by means of one-dimensional and two-dimensional electrophoresis (2D-PAGE), or prior digestion of a protein mixture (of the sample) with trypsin for subsequent peptide purification and analysis by means of mass spectrometry (MS), such as MALDI-TOF, or by means of multidimensional chromatography, by means of ICAT (isotope-coded affinity tags), DIGE (differential gel electrophoresis), or protein arrays.

The terms "polynucleotide" and "nucleic acid" are used interchangeably herein and refer to polymeric forms of nucleotides of any length, both ribonucleotides (RNA) and deoxyribonucleotides (DNA).

The terms "amino acid sequence," "peptide," "oligopeptide," "polypeptide," and "protein" are used interchangeably herein and refer to a polymeric form of amino acids of any length, which can be chemically or biochemically modified coding or non-coding amino acids.

Throughout the description and claims, the word "comprises" and variants thereof do not intend to exclude other technical features, additives, components, or steps. For those skilled in the art, other objects, advantages, and features of the invention will become apparent in part from the description and in part from the practice of the invention. The following examples and drawings are provided by way of illustration and are not intended to be limiting of the present invention.

### EXAMPLES OF THE INVENTION

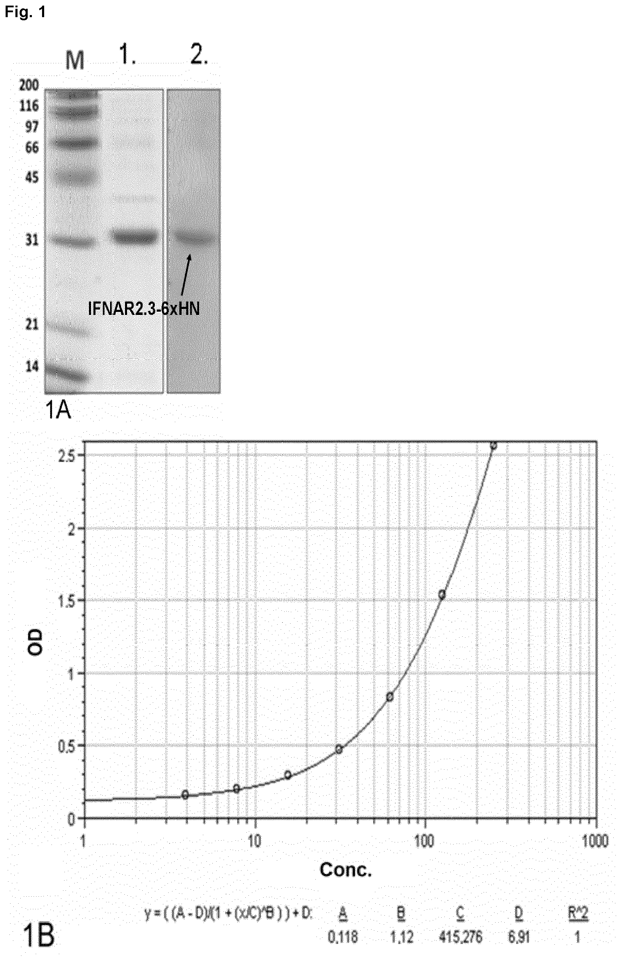

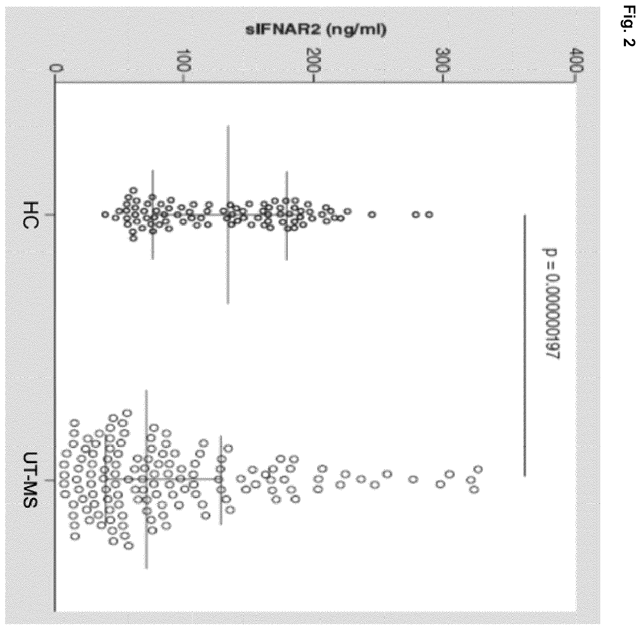

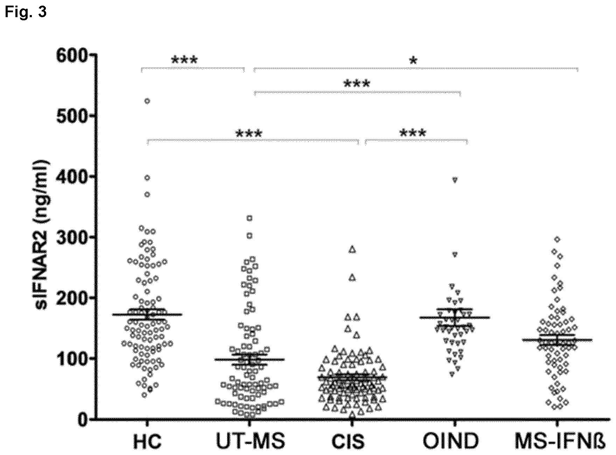

First, the authors of the invention cloned a recombinant protein analogous to human sIFNAR2, which was identified by means of WB and also by means of MALDI-TOF. This protein was used in the development and validation of an ELISA for detecting serum sIFNAR2 (Figure 1) (Órpez-Zafra T. Bioanalysis 2015; 2869-2880). Clinical implementation in two independent cohorts showed significantly lower levels in non-infarcted MS patients than in healthy controls (Figures 2 and 3) and increased levels in patients treated with IFNβ (Figure 3) (Órpez-Zafra T. Bioanalysis 2015; 2869-2880; Órpez-Zafra T. Mult Scler. 2017 Jun; 23(7): 937-945). It is a recombinant protein of sIFNAR2 with 249 amino acids and a molecular weight of 29 KDa, which corresponds to the soluble fraction of the IFNβ receptor. This protein has been used as a standard in the development and validation of an ELISA for detecting the soluble form of the IFNβ receptor (sIFNAR2) in serum.

A longitudinal study including 66 MS patients (baseline, 6 and 12 months after the start of treatment with IFN), with 51 classified as responders (R) and non-responders (NR) according to the Rio score (Rio J. Mult Scler. 2009. 15: 848-53), was performed. Flare ups, progression in the expanded disability status scale (EDSS), and MRI activity were considered as therapy response variables. Twenty-three patients were considered non-responders according to the occurrence of two or three positive variables during the first year of therapy, in contrast 28 were considered responders. Furthermore, 12 MS patients were included in the moment of flare up and then in remission. For the clinical form, 143 relapsing-remitting, 43 secondary progressive, and 12 primary progressive patients were analyzed.

Serum slFNAR2 was quantified by means of an ELISA developed and validated in the laboratory of the present authors (Órpez-Zafra T. Bioanalysis 2015; 2869-2880). Each assay included a standard curve, 2 quality controls, and a negative control. The concentration of sIFNAR2 was determined by means of the optical interpolation of the samples and controls on the standard curve. The calibration curve was established using a four-parameter curve fitting model. Non-parametric tests were used to compare sIFNAR2 levels between groups.

It was observed that before the start of treatment, NR patients had significantly lower slFNAR2 levels in comparison with R patients (p = 0.026). Logistic regression analysis showed that patients with baseline slFNAR2 levels below 43.2 µg/ml have an OR of 5.1 (p = 0.012 IC [1.42-18.25]) of non-responders to treatment with IFNβ. The model was fitted for possible variables of confusion, such as sex, age, and time of evolution. Therefore, individuals having baseline sIFNAR2 levels below 100 µg/ml, and more preferably below 90, 80, 70, 60, 50, and even more preferably 45 µg/ml, can be included in the group of individuals with multiple sclerosis not responding to treatment with IFNβ.

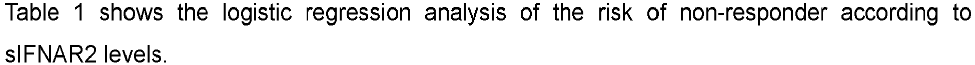

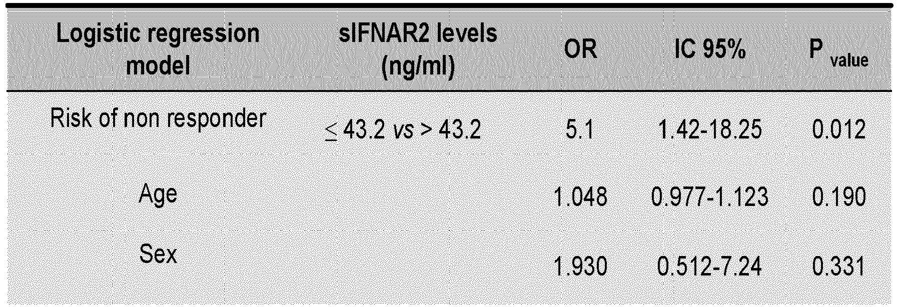

The determination of slFNAR2 by means of ELISA could be used in clinical practice as a biomarker for predicting whether a patient will respond to treatment with IFNβ. The ELISA technique is easy to implement in clinical diagnostic laboratories, and since determination is performed in serum, the method is minimally invasive for the patient.

## Claims

1. A method for predicting or prognosticating the response of an individual with multiple sclerosis to treatment with IFNβ which comprises:
a) measuring slFNAR2 protein levels in a serum sample isolated from the individual, and
b) comparing the levels obtained in step (a) with a reference amount, and
c) assigning the individual of step (a) to the group of non-responder individuals when he or she has significantly lower slFNAR2 protein levels in serum in comparison with responder individuals.

2. The method according to claim 1, wherein the determination of sIFNAR2 levels is performed by means of an immunoassay.

3. The method according to any of claims 1 or 2, wherein the determination of slFNAR2 levels is performed by means of a technique selected from: immunoblot, enzyme-linked immunosorbent assay (ELISA), line immunoassay (LIA), radioimmunoassay (RIA), immunofluorescence, or protein chips.

4. The method according to any of claims 1 to 3, wherein the determination of slFNAR2 levels is performed by means of an enzyme-linked immunosorbent assay or ELISA.

5. The method according to any of claims 1 to 4, wherein the determination of slFNAR2 levels is performed by means of a sandwich ELISA.

6. Use of a kit comprising the elements needed for quantifying sIFNAR2 protein levels in serum, for predicting or prognosticating the response of an individual with multiple sclerosis to treatment with IFNβ;
wherein said kit comprises at least one anti- slFNAR2 antibody.

7. The use of a kit according to the preceding claim, comprising a primary antibody comprising the amino acid sequence of SEQ ID NO: 3.

8. The use of a kit according to any of claims 6 or 7, comprising a secondary antibody comprising the amino acid sequence of SEQ ID NO: 4.

9. The use of a kit according to any of claims 6 to 8, comprising a recombinant protein having an amino acid sequence of SEQ ID NO: 2.

10. The use of a kit according to any of claims 6 to 9, further comprising:
a) a solid support with a primary antibody attached thereto;
b) a secondary antibody,
c) a solution containing an enzymatic marker-labeled detection antibody; and
d) a reagent.

## Patentansprüche

1. Verfahren zur Vorhersage oder Prognostizierung des Ansprechens eines Individuums mit multipler Sklerose auf die Behandlung mit IFNβ, welches Folgendes umfasst:
a) das Messen von slFNAR2-Proteingehalten in einer Serumprobe isoliert aus dem Individuum, und
b) das Vergleichen der in Schritt (a) erhaltenen Gehalten mit einer Referenzmenge, und
c) das Zuordnen des Individuum aus Schritt (a) einer Gruppe von Non-Responder-Individuen, wenn er oder sie signifikant niedrigere slFNAR2-Proteingehalten in Serum im Vergleich zu Responder-Individuen aufweist.

2. Verfahren nach Anspruch 1, wobei die Bestimmung von slFNAR2-Gehalten mittels eines Immunassays durchgeführt wird.

3. Verfahren nach einem der Ansprüche 1 oder 2, wobei die Bestimmung von slFNAR2-Gehalten mittels einer Technik ausgewählt aus: Immunblot, enzymverbundenem Immun-Sorptions-Versuch (ELISA), Line-Immunassay (LIA), Radioimmuntest (RIA), Immunfluoreszenz oder Protein-Chips durchgeführt wird.

4. Verfahren nach einem der Ansprüche 1 bis 3, wobei die Bestimmung von slFNAR2-Gehalten mittels eines enzymverbundenen Immun-Sorptions-Versuches oder ELISA-Tests durchgeführt wird.

5. Verfahren nach einem der Ansprüche 1 bis 4, wobei die Bestimmung von slFNAR2-Gehalten mittels eines Sandwich-ELISA-Tests durchgeführt wird.

6. Verwendung eines Kits umfassend die nötigen Elemente zur Quantifizierung von slFNAR2-Proteingehalten in Serum, zur Vorhersage oder Prognostizierung des Ansprechens eines Individuums mit multipler Sklerose auf die Behandlung mit IFNβ;
wobei das genannte Kit mindestens einen anti-slFNAR2 Antikörper umfasst.

7. Verwendung eines Kits nach dem vorhergehenden Anspruch, umfassend einen primären Antikörper, welcher die Aminosäuresequenz aus SEQ ID NO: 3 umfasst.

8. Verwendung eines Kits nach einem der Ansprüche 6 oder 7, umfassend einen sekundären Antikörper, welcher die Aminosäuresequenz aus SEQ ID NO: 4 umfasst.

9. Verwendung eines Kits nach einem der Ansprüche 6 bis 8, umfassend ein rekombinantes Protein, welches eine Aminosäuresequenz aus SEQ ID NO: 2 aufweist.

10. Verwendung eines Kits nach einem der Ansprüche 6 bis 9, zusätzlich umfassend:
a) einen festen Träger mit einem daran befestigten primären Antikörper;
b) einen sekundären Antikörper,
c) eine Lösung enthaltend einen mit einem enzymatischem Marker markierten Detektionsantikörper; und
d) ein Reagens.

## Revendications

1. Méthode pour prédire ou pronostiquer la réponse d'un individu avec sclérose en plaques au traitement avec l'IFNβ, qui comprend :
a) mesurer des niveaux de protéine sIFNAR2 dans un échantillon de sérum isolé de l'individu, et
b) comparer les niveaux obtenus dans l'étape (a) avec une quantité de référence, et
c) assigner l'individu de l'étape (a) au groupe des individus non-répondeurs lorsqu'il ou elle a des niveaux de protéine sIFNAR2 significativement inférieurs en sérum en comparaison avec des individus répondeurs.

2. Méthode selon la revendication 1, dans laquelle la détermination de niveaux de siFNAR2 est effectuée au moyen d'un immunoessai.

3. Méthode selon l'une quelconque des revendications 1 ou 2, dans laquelle la détermination de niveaux de sIFNAR2 est effectuée au moyen d'une technique sélectionnée parmi : immunoblot, test immunoenzymatique (ELISA), immunoessai en ligne (LIA), radioimmunoessai (RIA), immunofluorescence ou puces à protéines

4. Méthode selon l'une quelconque des revendications 1 à 3, dans laquelle la détermination de niveaux de sIFNAR2 est effectuée au moyen d'un test immunoenzymatique ou ELISA.

5. Méthode selon l'une quelconque des revendications 1 à 4, dans laquelle la détermination de niveaux de sIFNAR2 est effectuée au moyen d'un ELISA de type sandwich.

6. Utilisation d'un kit comprenant les éléments nécessaires pour quantifier des niveaux de protéines sIFNAR2 en sérum, pour prédire ou pronostiquer la réponse d'un individu avec sclérose en plaques au traitement avec IFNβ ;
dans laquelle ledit kit comprend au moins un anticorps anti-sIFNAR2.

7. Utilisation d'un kit selon la revendication précédente, comprenant un anticorps primaire comprenant la séquence d'acides aminés de SEQ ID NO : 3.

8. Utilisation d'un kit selon l'une quelconque des revendications 6 ou 7, comprenant un anticorps secondaire comprenant la séquence d'acides aminés de SEQ ID NO : 4.

9. Utilisation d'un kit selon l'une quelconque des revendications 6 à 8, comprenant une protéine recombinante ayant une séquence d'acides aminés de SEQ ID NO : 2

10. Utilisation d'un kit selon l'une quelconque des revendications 6 à 9, comprenant en outre :
a) un support solide avec un anticorps primaire fixé à celui-ci ;
b) un anticorps secondaire,
c) une solution contenant un anticorps de détection marqué par un marqueur enzymatique ; et
d) un réactif.
